(19) Europäisches Patentamt
European Patent Office
Office européen des brevets

(11) Publication number: **0 190 480**
**B1**

(12) # EUROPEAN PATENT SPECIFICATION

(45) Date of publication of patent specification: **02.05.90**

(51) Int. Cl.$^5$: **C 01 B 33/22**, C 01 B 33/34, B 01 J 29/28, C 07 C 2/66

(21) Application number: **85300770.6**

(22) Date of filing: **05.02.85**

(54) **Magnesium silicate compositions, process for their preparation and alkylation process employing same.**

(43) Date of publication of application:
**13.08.86 Bulletin 86/33**

(45) Publication of the grant of the patent:
**02.05.90 Bulletin 90/18**

(84) Designated Contracting States:
**BE DE FR GB IT NL**

(56) References cited:
**EP-A-0 014 545**
**EP-A-0 104 800**
**EP-A-0 113 234**
**GB-A-1 449 129**
**US-A-2 384 563**
**US-A-4 117 020**

(73) Proprietor: **THE DOW CHEMICAL COMPANY**
**2030 Dow Center Abbott Road P.O. Box 1967**
**Midland Michigan 48640-1967 (US)**

(72) Inventor: **Garces, Juan M.**
**5217 Cortland Street**
**Midland Michigan 48640 (US)**

(74) Representative: **Raynor, John et al**
**W.H. Beck, Greener & Co 7 Stone Buildings**
**Lincoln's Inn**
**London WC2A 3SZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 190 480 B1

## Description

The present invention relates to novel magnesium silicates and methods for their preparation. In particular, the present invention relates to novel magnesium silicates having a porous structure and catalytic properties.

In another aspect, the present invention relates to the alkylation of aromatic compounds by the use of novel magnesium silicates of the present invention. In particular, the present invention relates to the alkylation of benzene and alkylbenzenes or mixtures thereof by the use of novel magnesium silicates having a porous structure and catalystic properties.

Silicates are materials having as a fundamental unit a tetrahedral complex consisting of $Si^{4+}$ in tetrahedral coordination with four oxygens. Less common but also known is an octahedral complex consisting of $Si^{4+}$ in octahedral coordination with six oxygens. In some structures, the tetrahedra link to form chains or sheets. If the linking occurs in three dimensions, what is termed a framework structure results. Framework silicates may have substituted elements in place of some of the silicon atoms. A common substitution is that of aluminum for silicon forming what are known as aluminosilicates.

Zeolites are typically crystalline, hydrated aluminosilicates of Group I or Group II elements as formed in nature or synthesized. Structurally the zeolites are porous crystalline framework aluminosilicates which are based on an extending three-dimensional network of $SiO_4$ and $AlO_4$ tetrahedra linked to each other by shared oxygens.

Molecular sieves are compositions which have the property of acting as sieves on a molecular scale. Dehydrated crystalline zeolites are important molecular sieves. Zeolites, as well as coals, oxides, glasses, intercalation compounds of graphite and alkali metals, pillared clays, and carbon absorbents are types of molecular sieves. Classification and identification of the aforementioned compositions is difficult with varied and inconsistent usage of terms found in the literature. For further information and background regarding molecular sieves and zeolites see Breck, *Zeolite Molecular Sieves*, John Wiley & Sons (1974).

Molecular sieves in general and zeolite catalysts in particular have found widespread industrial use. From a catalytic viewpoint, zeolites are molecular sieves having molecular cages with variable dimensions. Zeolite catalysts have the important properties of (1) ingress/egress control of molecules, (2) high density of "active" sites, (3) sites for occluded or deposited chemical moieties such as salts or metals, and (4) controllable potential energy fields. Zeolites are useful as catalysts in such processes as catalytic cracking, conversion of methanol to gasoline, polymerization reactions, and alkylating aromatics to mention a few. Also, molecular sieves find such utilities as acting as selective absorbents or facilitating separation of isomers.

Zeolite molecular sieves are found in nature and have been known for many years. The most siliceous natural zeolites are ferrierite, clinoptilolite and mordenite all having a $SiO_2/Al_2O_3$ ratio of 10:1. Synthetic zeolites were first commercially produced with the introduction of zeolite A around 1954. The first synthetic zeolites had $SiO_2/Al_2O_3$ ratios in the range of 2:1 to 10:1. Later synthetic zeolites were made having molar ratios in excess of 10:1.

In 1972, US 3,702,886 (Argauer) issued for a synthetic zeolite termed ZSM-5 and method for making same. This patent disclosed a zeolite having a $SiO_2/Al_2O_3$ molar ratio from 5 to 100. The main claim characterized ZSM-5 by reference to a table of X-ray diffraction lines (see Table I infra) and the following composition in terms of molar ratios of oxides

$$0.9 \pm 0.2\ M_{2/n}O:Al_2O_3:YSiO_2:ZH_2O$$

wherein M is at least one cation having a valence n, Y is at least 5 and Z is between 0 and 40.

TABLE I

ZSM-5, Interplanar Spacing d(Å)

| | | | |
|---|---|---|---|
| 11.1±0.2 | 6.30±0.1 | 5.01±0.1 | 3.71±0.05 |
| 10.0±0.2 | 6.04 ±0.1 | 4.60±0.08 | 3.04±0.03 |
| 7.4±0.15 | 5.97 | 4.25±0.08 | 2.99±0.02 |
| 7.1±0.15 | 5.56±0.1 | 3.85±0.07 | 2.94±0.02 |

The ZSM—5 aluminosilicate is prepared by including nitrogenous organic molecules such as tetrapropyl ammonium bromide in the reaction mixtures. For very high $SiO_2/Al_2O_3$ preparations, no aluminum need be deliberately added since it is present as an impurity in the reactants. The organic molecules are incorporated into the framework structure as it forms and these as-synthesized materials are termed "nitrogenous zeolites". Application of high temperatures will free high $SiO_2/Al_2O_3$ materials of

these organic components without altering the basic framework structure, D. M. Olson et al., "Chemical and Physical Properties of the ZSM-5 Substitutional Series", *J. Catal., 61*, 390—396 at 391 (1980).

It is known that many properties of zeolites are primarily dependent upon the framework structure while remaining essentially invariant with composition changes such as altering the $SiO_2/Al_2O_3$ ratio. These properties include the X-ray diffraction pattern, pore size and volume, framework density and refractive index. Other properties of zeolites such as ZSM-5 will vary with composition. These properties include catalytic activity, ion-exchange capacity, and hydrophobicity (see Olson, *supra*, at 391).

A process for alteration of the $SiO_2/Al_2O_3$ ratio as well as zeolite composition in general was disclosed in 1973 in US 3,761,396 (Pickert II). This patent reviews means for removing aluminum from aluminosilciates to produce more siliceous materials. This patent also reports that in some instances, aluminum in the zeolitic crystalline structure can be substituted by other metals. In a related earlier patent US 3,640,681 (Pickert I) which issued in 1972, the extraction of framework aluminum from large pore crystalline zeolitic molecular sieves is examined in detail. A process is claimed which utilizes acetylacetonate as an agent for aluminum removal. Also when used with a metal acetylacetonate, the metal can be substituted in the framework for the aluminum. Examples of such substitution are given which incorporate vanadium and chromium into Y zeolites. Metal acetylacetonates are preferred which utilize metals that form oxides in tri-, tetra- or pentavalent states and which are thermally stable at 600°C. However, mono- and divalent metals can also be used according to the written description which lists $Mg^{++}$ as being among those metals suitable for substitution.

US 4,049,573 (Kaeding) issued in 1977 disclosed a class of zeolites exemplified by ZSM-5, ZSM-11, ZSM-12, ZSM-35, and ZSM-38 whose members may be treated by impregnation with magnesium, boron or phosphorus compounds. These zeolites are catalysts having occluded or deposited metal moieties. These modified zeolites are useful as catalysts in hydrocarbon conversion reactions.

US 4,061,724 (Grose) issued in 1977 for a crystalline silica composition known as "silicalite" and method for preparing same. This composition has many molecular sieve properties similar to porous crystalline aluminosilicates, but does not have ion-exchange properties which are essential to zeolitic molecular sieves. Furthermore, the silicalite does not contain $AlO_4$ tetrahedra as essential framework constituents. Silicalite has an X-ray diffraction pattern very similar to the pattern for ZSM-5.

A detailed comparison of silicalite and ZSM-5 which discloses that both silicates are members of the same structural family is presented in Olson, D. H. et al., "Chemical and Physical Properties of the ZSM-5 Substitutional Series", *J. Catal., 61*, 390—396 (1980).

US 4,108,881 (Rollman I) discloses an aluminosilicate zeolite termed ZSM-11. The structure of ZSM-11 has been related to that of ZSM-5 and to that of silicalite in an article by G. T. Kokotailo and W. M. Meier, "Pentasil Family of High Silica Crystalline Materials", *Spec. Publ.-Chem. Soc., 33* (Prop. Appl. Zeolites), 133—139 (1980). This article describes the common structural features of the above materials and proposes a generic name — pentasil — to denominate this family of structures.

US 4,148,713 (Rollman II) discloses ZSM-5 particles having aluminum-free outer shells of $SiO_2$.

Within the group of framework silicates with tridimensional structure there is a plethora of examples which illustrate substitution of $Si^{4+}$ by metal ions other than $Al^{3+}$, as described by W. Eitel in the well-known book collection *Silicate Science*, Academic Press, 1964.

The substitution of $Si^{4+}$ by $Al^{3+}$ is common in framework silicates, but other metal ions are frequently found as substituents as well. For example, iron substitution is widespread in natural zeolites and in natural framework silicates.

Less common is the substitution of $Si^{4+}$ by divalent ions. In an article by H. Y-P Hong, "New Solid Electrolytes", *Adv. Chem. Ser., 163*, 179—194, A.C.S. 1977, the substitution of $Si^{4+}$ by divalent ions including magnesium in framework silicates is described. H. Y-P Hong distinguishes the properties of the resulting silicates in terms of the specific cations required to balance the electronic charge of the framework.

Recently publications have issued disclosing materials resembling zeolites in crystalline structure, but not being aluminosilicates. The framework atoms of these typically pentasil structured materials contain combinations of elements other than the usual list of aluminum, silicon, oxygen, gallium or germanium.

In European Patent Office document 3,662 (Maas et al.) published in January of 1979, the applicant, Shell International Research, discloses a process for the preparation and separation of organic isomers using crystalline silicates which contain iron atoms within the framework structure. These silicates have a pentasil structure similar to that of ZSM-5.

In United Kingdom Patent applications 2,023,562A (Taramasso I) published January 3, 1980 and 2,024,790A (Taramaso II) published January 16, 1980, applicant, Snamprogetti S.p.A., discloses materials giving X-ray diffraction patterns akin to ZSM-5. In the process of forming these materials, aminoalcohols, such as triethanolamine may be used. The materials formed may be used as catalysts. In addition to containing oxygen and silicon as part of the framework structure, these silicates may also include boron, beryllium, chromium, zinc, titanium, vanadium, manganese, iron, cobalt, zirconium, rhodium, silver or antimony.

In German patent document 2830787 (Marosi) published January 23, 1980, applicant, BASF, discloses a method for the production of nitrogen-containing crystalline metal silicates with zeolite structure. This document also discusses the framework substitution of metals in place of aluminum. Boron, arsenic,

antimony, vanadium, iron or chromium are given as examples of metals having a valence of three which may be substituted into the framework structure. It also states that elements such as chromium, vanadium and arsenic may be partially framework-substituted and partially deposited in the intracrystalline pores. These new materials are pentasil structured having a ZSM-5 or ZSM-8 structure in particular. A method of making this material is disclosed which utilizes hexamethylenediamine.

In United Kingdom patent document 2,033,358A (Morrison), published May 21, 1980, applicant, Mobil, discloses crystalline materials having ZSM-5 zeolite structures whose composition has silica to aluminum ratios ranging from 35 to 3000 or more. The composition of these materials may include rare earth metals, chromium, vanadium, moleybdenum, indium, boron, mercury, tellurium, silver, ruthenium, platinum or palladium, however, it is unclear how these elements are related in the composition.

In European Patent Office document 13,630 (Rosinski), published July 23, 1980, applicant, Mobil, discloses a ZSM-12 type material which contains chromium or iron or both with the chromium and iron believed to be in positions of tetrahedral-substitution within the silica lattice.

Mobil has also disclosed in European Patent Office document 14,545 (Chu et al.), published January 24, 1980, that the ZSM-5 class of zeolite catalysts are shape selective and that this shape selectivity can be enhanced by impregnation with magnesium or phosphorus to reduce zeolite pore openings as well as by the use of very large crystals and coke selectivation. This document further discloses impregnating or ion-exchanging a zeolite with metal-containing salts such as Mg, followed by high temperature calcination at 649°C or higher. In the examples, a solid-state exothermic reaction of magnesium impregnated ZSM-5 is reported at 871°C with the reaction believed to be between magnesium and ZSM-5.

US 4,229,424 (Kokotailo), discloses a crystalline zeolite product having a structure intermediate of that of ZSM-5 and ZSM-11.

The present invention is a novel porous crystalline magnesium silicate. The amount of magnesium present in this silicate may vary. However, for all compositions of the present invention, it is essential that some magnesium which is not ion-exchangeable by conventional techniques be present in the silicate. Conventional techniques of ion-exchange are presented in Breck, *Zeolite Molecular Sieves*, John Wiley & Sons (1974). Other elements may be present in these novel silicates as impurities such as aluminum, germanium, gallium, etc., or chemicals may be deliberately added either to modify or improve the properties of the magnesium silicate or for other advantageous reasons, for example, to ameliorate process parameters.

The novel silicates have a composition which may be expressed according to the following formula in terms of the molar ratios of oxides on a dry basis:

$$(M_{2/n}O)_p(MgO)_x(R_2O_3)_y(SiO_2)_z \qquad \text{Formula I}$$

wherein M is at least one ion-exchangeable cation having a valence of *n*; R is at least one element (with valence $3^+$) p is from 0.1 to 20; x is from 0.1 to 12; y is from 0 to 3; z is from 84 to 96 and p/n is greater than y, further characterized by

1. an infrared spectrum exhibiting at least two peaks in the 1200—980 $cm^{-1}$ region,
2. non-ion-exchangeable magnesium contained in the lattice, and
3. an X-ray diffraction trace having at least interplanar d spacings of 11.2 ± 0.2, 10.1 ± 0.2, 10.0 ± 0.2, 9.8 ± 0.2, 6.0 ± 0.2, 5.8 ± 0.2, 5.6 ± 0.2, 4.26 ± 0.1, 3.85 ± 0.05, 3.81 ± 0.05, 3.74 ± 0.03, 3.72 ± 0.03, 3.64 ± 0.03. By dry basis is meant material which has been heated in air at 500°C for a period of one hour or more. The invention is not limited to such dried material or said oxide forms, rather its composition may be presented in terms of oxides and on a dry basis (as in the above formula) in order to provide a means for identifying some of the novel compositions. In the above-mentioned formula, element R need not be present. Other formulas may be written by those skilled in the art to identify particular subsets or embodiments of the present invention which comprises porous crystalline magnesium silicates.

Preferred novel silicates have a composition which may be expressed according to the following formula in terms of molar ratios of oxides on a dry basis:

$$(Na_2O)_p(MgO)_x(SiO_2)_z \qquad \text{Formula II}$$

this y of formula I being zero.

Magnesium silicates of the present invention have useful properties including catalytic activity. These novel compositions may be advantageously employed in known processes which presently use zeolites or compositions having zeolitic or molecular sieve properties.

Compositions of the present invention may be advantageously incorporated with binders or other materials which are well-known in the art. They may also be modified with one or more elements or compounds by deposition, occlusion, ion-exchange or other techniques known to those skilled in the art to enhance, supplement or alter the properties or usefulness of the composition. See, e.g., Breck, *Zeolite Molecular Sieves*, John Wiley & Sons (1974).

The magnesium silicates of the present invention are prepared by hydrothermal methods from a variety of silicate and magnesium sources leading to products of this invention, all of which incorporate magnesium into the structure of the resulting crystalline silicate.

As mentioned above, the novel compositions presented have properties akin to aluminosilicate zeolites and other silicates. These properties result from the particular crystalline structure and composition of these silicates. A major problem plaguing past and current research concerning porous silicates is how to properly characterize newly synthesized materials. In "Zeolite Molecular Sieves", pages 22—26, Breck discusses at length some of the vagaries of the nomenclature employed as well as problems associated with proper characterization. Clearly the proper characterization of a previously unknown material requires structural, compositional and physicochemical information. In the past the primary criteria for characterization, particularly in the patent literature has been (1) one or more powder X-ray diffraction patterns given to define the structure and (2) molar ratios of metal and organic oxides to define the composition. Sometimes in addition to the above, physicochemical properties such as the refractive index or absorption capacities for particular compounds or a ratio of cracking rate constants for two hydrocarbons are employed.

As progress continues in synthesizing new porous silicates with the concurrent realization that subtle changes in a material's composition and/or structure can produce significant differences in usefulness, it becomes clear that prior attempts at characterizing new materials are often inadequate. This frequent inability to adequately define or characterize newly synthesized materials properly, reflects the present skill of the art. Just as welding is often said to be more of an art than a science so too is the making of zeolites and zeolite-like materials. For example, it is known that often structural changes occur in some known zeolites merely by changing the order in which precursor materials are added or by altering stirring conditions such as the rate of stirring, the length of the mixing time or at which point in time in the process stirring commences and ends. Also with the discovery of new materials having various elements purportedly located as part of the framework structure, it becomes desirable to be able to relate composition to structure. A primary question concerning a new porous silicate containing an element Z, is the location of that element Z. Is it part of the lattice (framework structure) or is it occluded or deposited within a pore formed by the framework or is it electronically connected like those moieties which are ion-exchangeable? Merely giving a relationship between molar ratios of metal and/or organic oxides does not provide this important information and unfortunately there is no easy single means of analysis which will suffice. The skilled artisan has great difficulty in designating the location of element Z and such designations are usually made with much faith and inconclusive proof. The location of a given element in a new composition is complicated by the presence of more than one phase in the end product of many zeolite synthesis. In some instances what appears to be a single crystalline phase is actually a mixture of closely related structures as is the case with synthetic erionite-offretite mixtures. Besides the crystalline phases detected by X-ray diffraction in the end product, one finds amorphous material mixed with the crystalline zeolites.

Statements about the location of a given element in the crystalline phases should be based on characterization of the crystalline fraction of the products of synthesis.

From a practical point of view, the mixtures of crystalline phases and amorphous materials could offer specific advantages to the user in specific applications. For this reason, it is not essential to work with purified crystals in many applications. In fact, zeolites are even diluted with binders and modifiers for certain applications.

The end products of the synthesis of this invention are known to contain mainly crystalline material but they also contain amorphous silicates of magnesium. In the present invention, it is believed that essentially some magnesium is a part of the crystalline silicate structure. This essential portion of magensium may be thought of as being a part of the structure in the same sense that silicon or oxygen is a part of the structure.

The term crystalline when used in this document refers to materials which are recognized by those skilled in the art as having a highly ordered structure. Three dimensional periodicity is characteristic of a highly ordered structure. The skilled artisan recognizes that evidence of such periodicity may be presented by catalytic reactivity, infrared spectroscopy or other means of analysis as well as by the commonplace X-ray diffraction analysis. Silicates of the present invention are "crystalline" as that term is characterized above even if said silicates appear amorphous to X-ray diffraction analysis if a skilled artisan recognizes a highly ordered structure by other evidence. See, e.g., P. A. Jacobs et al., "Evidence of X-ray Amorphous Zeolites", *J.C.S. Chem. Comm.*, 591, 1981.

By the term "porous" are meant those silicates having a framework structure containing cavities capable of allowing the entrance or absorbence of molecules such as water, nitrogen or toluene, etc.

Due to the differences in ionic of $Si^{IV}$ (0.41 Å) and $Al^{III}$ (0.50 Å) replacement of Si by Al in $TO_4$ sites will cause a unit cell volume expansion in most zeolites. The degree of unit cell volume expansion will depend on the amount of Al substitution for Si in the $TO_4$ sites. If the substitution is low, as in some ZSM-5 and silicalite zeolites, high resolution, calibrated X-ray diffraction techniques must be utilized to detect the expansion.

Similarly, in the present invention, it is believed that nonion-exchangeable Mg is contained in the zeolite lattice. Replacement of $Si^{IV}$ (0.41 Å) by $Mg^{II}$ (0.65 Å) in $TO_4$ sites will also cause a unit cell expansion. Once again, the amount of Mg substitution for Si, will influence the degree of cell volume expansion.

Evidencing element location in a framework lattice structure by determining cell volume expansion (contraction) has been done by others skilled in making silicates. See, e.g., M. Taramasso, G. Perego and B. Notari, "Molecular Sieve Borosilicates", *Proceedings of the Fifth International Conference on Zeolites,*

40—48 at 44 (Heyden & Sons Ltd.) (1980).

High resolution X-ray powder diffraction data were obtained from Huber-Guinier powder diffraction cameras equipped with Ge and quartz monochromators for providing $CuK_{\alpha 1}$ and $FeK_{\alpha 1}$ radiation, respectively. The films were calibrated, with well-known internal standards such as NBS Si (NBS Circular 539 *Vol. 9,* p. 3) or $As_2O_3$, scanned with a densitometer and the resulting data profile fit by techniques described in: J. W. Edmonds and W. W. Henslee, *Adv. in X-ray Anal., 22,* 143 (1978) and J. W. Edmonds, "Precision Guinier X-ray Powder Diffraction Data", NBS Special Publication 567, *Proceedings of Symposium on Accuracy in Powder Diffraction Held at NBS, Gaithersburg, MD,* June 11—15, 1979 (Issued February 1980). The calibrated data were least-squares refined and fitted to obtain accurate cell dimensions and volumes.

Using data from the method described above and using single crystal X-ray crystallographic data from the literature, the cell volume for the present invention where $Mg^{II}$ is believed to replace $Si^{IV}$, can be compared to the cell volume of silicalite which has $Si^{IV}$ in all the $TO_4$ sites. Typical data are shown in Table II, for either anhydrous zeolites or calcined zeolites. (Minimum calcination of 500°C for 1 hour.)

TABLE II

Cell Volumes

| Compound | Volume Å$^3$) | Reference |
|---|---|---|
| Silicalite | 5306 | 1 |
| Silicalite | 5305 | 2 |
| Magnesium Silicate | 5347 | 2 |
| Magnesium Silicate | 5349 | 2 |

[1] Cell volumes were obtained from the lattice parameters given in an article by E. M. Flanigen, J. M. Bennett, R. W. Grose, J. P. Cohen, R. L. Patton, R. M. Kirchner and J. V. Smith, *Nature, 271,* 512 (1978).

[2] Cell volumes were calculated using the National Bureau of Standards - Geological Survey Lattice Parameter Refinement Program written by Dan Appleman (available through NTIS) on XRD data obtained on samples made either according to a process of the invention or according to the silicalite patent, US 4,061,724.

The above values are typical examples of cell volumes. The difference between these volumes show a cell volume expansion. The exact amount of expansion will be composition dependent. The compounds of the present invention will exhibit unit cell volume expansion when compared to silicalite, but expansion is not limited to that derived from the data shown in Table II. It is believed that the above-mentioned unit cell expansion evidences the placement of magnesium as a part of the lattice framework structure. It is believed, without wishing to be bound by that belief, that altering the $SiO_2/MgO$ ratio varies the pore size and volume, framework density and refractive index of the resulting magnesium silicates. If small ranges of the $SiO_2/MgO$ ratios are utilized, the ability to detect volume, pore size and density differences will be dependent on the resolution capabilities of the analytical technique used.

Samples of compositions of the present invention whose crystallite size is appropriate to produce a distinct X-ray powder diffraction trace, have a pattern which includes at least the interplanar d spacings listed in Table III.

TABLE III

| Magnesium silicate, interplanar spacings d(Å) |
|---|
| 11.2 ±0.2 |
| 10.1 ±0.2 |
| 10.0 ±0.2 |
| 9.8 ±0.2 |
| 6.0 ±0.2 |
| 5.8 ±0.2 |
| 5.6 ±0.2 |
| 4.26±0.1 |
| 3.85±0.05 |
| 3.81±0.05 |
| 3.74±0.03 |
| 3.72±0.03 |
| 3.64±0.03 |

The range cited is due to unit cell volume expansion with decreasing $SiO_2/MgO$ ratio. Magnesium silicates with low Mg content in the $TO_4$ sites will be near the low d spacing limit and those with high Mg content in $TO_4$ sites will be near the high d spacing limit.

The compositions of this invention are further characterized by a minimum of two reflections at 10.1 ± 0.3 Å and a minimum of four reflections between 3.69 (generally 3.72) and 3.90 Å.

These values were obtained by Huber-Guinier techniques (preferred method) mentioned previously or by a Philips Electronics X-ray powder diffraction unit equipped with: scintillation-counter detector, graphite monochromator, and a strip chart recorder. The recorded reflections were identified by their two theta locations, after these locations were calibrated with an internal standard. The standard used was either NBS Si (NBS Circular 539, *Vol. 9*, p. 3) or $As_2O_3$. The magnesium silicate diffraction peaks at approximately 10.0 and 3.81 Å can often be obscured in poorly crystalline samples or in low-resolution X-ray diffraction data.

X-ray analyses of magnesium silicates of the present invention reveal distinct differences in the diffraction patterns as a result of specific treatments given to these magnesium silicates. Intensity changes are observed and lines may appear, disappear or merge depending on the exact calcination procedure utilized. Ion-exchange of these silicates may also cause changes in certain cases. Several authors have made similar observations on related materials like zeolite ZSM-5. See H. Nakamoto and H. Tarahashi, *Chem. Lett.* 1013—1016 (1981). Regardless of the causes of the above-mentioned changes, they are expected by those people skilled in the art of analyzing porous crystalline silicates.

The magnesium silicates of this invention are characterized also by infrared analysis. The use of infrared analysis is recognized as a standard method in the characterization of inorganic and organic materials and has been used in the study of both natural and synthetic zeolites. See for example, E. M. Flanigen et al., *Adv. Chem. Series,* Vol. 101, p. 201—229, 1971. See also P. A. Jacobs, *supra*. For examples from the patent literature pertaining to the use of infrared analysis in zeolite characterization, see US 4,257,885 to R. W. Grose and E. M. Flanigen and references included therein.

Magnesium silicates of the present invention exhibit unique features in the 1300—400 $cm^{-1}$ region. Many compositions of this invention exhibit at least two distinct bands in the 1200—980 $cm^{-1}$ region as shown in Figures 1, 2 and 3. Preferred compositions of the present invention exhibit these two distinct bands and also characteristic infrared bands at 1225 ± 10 $cm^{-1}$, 800 ± 20 $cm^{-1}$, 620 ± 10 $cm^{-1}$, 550 ± 20 $cm^{-1}$ and 450 ± 20 $cm^{-1}$ as shown in Figures 1, 2 and 3.

Without wishing to be bound by any theory, it should be recognized that bands located between 1200—980 $cm^{-1}$ may be due to asymmetric stretch of $TO_4$ units in zeolites and silicates, see, e.g., Flanigen et al. "Molecular Sieve Zeolites-1" *Adv. Chem. Series, 101,* 201 A.C.S. (1971). It is believed without being bound by that belief that the band found nearest to 980 $cm^{-1}$ in the present invention is due to silanol groups of the form $-Si(OH)_3$, $>Si(OH)_2$, $\equiv SiOH$, or to their corresponding silicate forms.

Differential thermal analysis (DTA) is one of the thermal methods used in the literature as an aid in zeolite characterization. See D. W. Breck, *Zeolites Molecular Sieves*, John Wiley, 1974. See also European Patent Office Document 14,545 (Chu et al.), January 24, 1980.

Compositions of the present invention may be analyzed by DTA methods. When using a Dupont® 990 thermal analysis unit equipped with a 1200°C furnace, a 10 mg sample is tested against alumina as a reference material (both contained in platinum crucibles). The heating rate for the system is 20°C per minute in air with an air flow rate of 50 $cm^3$ per minute. Under these conditions, one observes a distinct exotherm at 870 ± 30°C. X-ray diffraction (XRD) analysis of the sample both before and after the exotherm yields at least the interplanar d spacings listed in Table III, *supra*.

The compositions of this invention have ion-exchange properties. The ion-exchange capacity of traditional zeolites is associated with their aluminum content. The ion-exchange properties of the magnesium silicate of this invention are not necessarily dependent upon any one of its particular components. Indeed it is believed, without wishing to be bound to this belief, that the ion-exchange capacity of the present invention is due to a combination of factors. Among them are: the magnesium content, the trivalent metal ion content and also to the presence of internal silanol moieties within the silicate framework which under appropriate conditions can participate in the ion-exchange process.

Even though a relationship among the composition and the ion-exchange capacity of these solids is recognized, the present invention is not restricted by the traditional "linear relationship" between composition and ion-exchange capacity, characteristic of traditional zeolites.

The exchangeable cations in zeolite compositions often play a critical role in their synthesis by hydrothermal methods. In certain cases, a particular cation is required to obtain a given zeolite, for example, sodium is said to be required to produce zeolite X from alminosilicate gels. Apparently the cation plays a template role in the formation of certain structures and/or acts as a crystallization promoter. The magnesium silicates of this invention do not appear to require a particular alkali metal cation for their formation. Crystalline compositions of the present invention are obtained from magnesium silicate gels in the presence of several alkaline metal salts including sodium or potassium salts. The presence of sodium or potassium ions during and/or after the synthesis may affect certain properties of the final product in applications which are susceptible to drastic changes by subtle differences such as catalysis and adsorption. Salts other than sodium and potassium may have similar effects.

In the synthesis of traditional zeolites the source of silica may be a critical factor in the preparation of certain zeolites. In the case of the present invention, the source of silica appears to have an effect in the morphology of the crystalline product. There are many examples in the literature relating morphology to a variety of useful properties of porous crystalline silicates like catalytic applications, ion-exchange, adsorption, etc.

Typically, the novel composition is made by hydrothermal methods using one of many sources of silicon such as one of the commercially available soluble silicates or water glass solutions, amorphous silica, colloidal silica, silica gels, fumed silica or an organosilicate like $(EtO)_4Si$. Advantageously employed are two commercially available sources: a colloidal silica sold by the du Pont de Nemours Company under the trademark Ludox SM® and a sodium silicate sold by the Philadelphia Quartz Company under the trademark Philadelphia Quartz Sodium Silicate N®.

The source of magnesium usually is one of its water-soluble salts, magnesium chloride, acetate, sulfate, nitrate, etc., or a complex ion like $Mg(NH_3)_6^{2+}$, $Mg(EDTA)^{2-}$, etc., or a slightly soluble compound like $Mg(OH)_2$, $MgF_2$, etc. A magnesium chloride salt is a preferred source of magnesium.

Besides these components the reaction mixture will contain a solvent such as water, along with alkali metal ion salts such as, chlorides, sulfates or hydroxides of sodium, potassium, rubidium or cesium. The solvent may be added separately to the reaction mixture or may already be present with one of the reactants such as the silica source. Water is the preferred solvent.

A material which is believed, without wishing to be bound by that belief, to act as a crystallization promoter and is hereinafter termed a "crystallization promoter" is utilized in the process of making the porous crystalline magnesium silicate of the present invention. Typically, this crystallization promoter is (or is formed from) an organic nitrogen compound such as quaternary ammonium ion salts, or hexamethylene diamine, but may also be other compounds such as seed crystals typically of compositions similar to those crystals sought from the process. In particular, tetrapropyl ammonium ion salts are often used with tetrapropyl ammonium bromide and tetrapropyl ammonium hydroxide being preferred.

In a typical method of making these novel magnesium silicates, a magnesium source, a crystallization promoter, an alkali metal ion salt and a solvent are combined. The pH of this combination of chemicals is usually adjusted and the combination is further combined with a mixture of a silicon source and a solvent to give a reaction mixture typically having a pH of 11. The H may advantageously be adjusted either above or below a pH of 11 to modify certain crystallization or process parameters such as the solubility of magnesium in the mixture, formation of precipitates, rates of crystallization, etc. The pH is adjusted as desired using acids or bases such as $H_2SO_4$ or NaOH and may be adjusted before, after and/or during the mixing step of the reactants.

The reaction mixture is vigorously mixed at room temperature for a sufficient time to produce an apparently homogeneous gel. Typically the rate of mixing is sufficiently vigorous to produce a satisfactory slurry or gel within one minute.

The mixture resulting from the above procedure is allowed to crystallize into compositions of the present invention. Preferably, crystallization takes place at temperatures above room temperature to increase the rate of crystal growth. More preferably, crystallization takes place at temperatures from 80°C to

200°C. Usually temperatures from 125°C to 150°C are used with autogeneous pressure. Higher or lower temperatures may be advantageously employed depending upon the process or product parameters desired, e.g., larger crystals are generally formed with lower temperatures and the rate of crystallization increases with higher temperatures. When quaternary ammonium ion salts are used as crystallization promoters, temperatures above 200°C are avoided to prevent their decomposition.

Crystallization is allowed to proceed until crystals of the compositions of the present invention are formed. This may be determined by analysis of reaction mixture samples at intervals. The crystallization time will vary depending upon the reactants or the particular process parameters chosen. Crystallization times of one to five days are not uncommon.

During the crystallization step, stirring may be advantageously employed to facilitate product formation. The rate and type of stirring may affect crystallization parameters such as the rate of crystallization, uniformity of the product and crystal size. The effect of this parameter and optimum adjustment is dependent upon other parameters and is believed to be within the skill of the art to determine without undue experimentation.

Following crystallization it is often desirable to filter the crystallized mixture using a water wash to remove the mother liquor and then to heat the crystals to 110°C to remove water and thereby produce a convenient free-flowing powder.

The compositions as made by the above procedure may contain organic moieties which, if desired, may be removed by known methods such as calcination in an oxygen-containing atmosphere at a temperature sufficient to decompose the organic moieties. Calcination at 500°C—600°C for approximately an hour is sufficient to remove commonly present organic moieties.

As mentioned before, the magnesium silicates of the invention may be beneficially modified by techniques well-known in the art which treat said silicates with acids, salts or other ions or molecules. Acid treatment is especially valued to produce a stable, catalytically active form of porous crystalline magnesium silicate. As is known in the art, the acid form or hydrogen form of such compositions may be prepared by contacting with an ammonium salt solution followed by drying and calcining. Alternatively, the composition may be acid-exchanged by contact with an acid solution such as hydrochloric acid.

As mentioned before, compositions of the invention may be expressed according to a formula in terms of the molar ratios of oxides on a dry basis, viz.,

$$(M_{2/n}O)_p(MgO)_x(R_2O_3)_y(SiO_2)_z$$

The moiety ($R_2O_3$) containing at least one element R with valence 3+ which is not ion-exchangeable by conventional means is a nonessential one. Typical nonion-exchangeable elements which may advantageously be present include by way of example, aluminum, iron, chronium, boron and gallium.

A preferred composition employed as a catalyst in the alkylation process of the present invention is also one that has been phosphorous treated in known manner to achieve selective isomer formation.

The products of the present invention expressed in terms of Formula I are those wherein p is from 0.1 to 20: x is from 0.1 to 12; y is from 0 to 3; and z is from 84 to 96. Further preferred embodiments are those wherein p is from 0.1 to 4, most preferably from 0.1 to 2.0; x is from 0.01 to 2.0, most preferably from 0.01 to about 0.5; y is from 0 to 3; and z is from 90 to 96. It is especially preferred that the term y of the above formula be from 0 to 1.0.

Typically, the ion-exchangeable cations M (of both the magnesium silicates represented by the above formula and similar magnesium silicates of the present invention) are alkali metals, hydrogen, group VIII metals or rare earth elements, or ammonium ions, but may be any element or moiety capable of exchange into the magnesium silicates of the present invention. Preferred are hydrogen, the alkali metals and the rare earth elements. Methods of ion-exchange are well-known in the art, e.g., hydrogen may be exchanged into a silicate by simply treating with acid.

The present invention also relates to the alkylation of aromatic compounds by the use of novel magnesium silicates. In particular, the present invention relates to the alkylation of benzene and alkylbenzenes or mixtures thereof by the use of novel magnesium silicates having a porous structure and catalytic properties.

Processes for the alkylation of aromatic compounds and particularly, processes for the ethylation of toluene are well-known, see e.g., US 4,086,287, and references cited therein. Catalysts for the reaction include those generally referred to as zeolites such as the ZSM-5 type zeolite catalysts and similar crystalline aluminosilicates such as those disclosed in the above US 4,086,287.

While the above noted prior art is considered of interest in connection with the subject matter of the instant application, the alkylation process described herein employs previously unknown porous magnesium silicate catalysts. Advantageously, the present process employs wide extremes of temperature and reactant ratios, and has not, as far as is known, been heretobefore described.

The present invention is an alkylation process employing as a catalyst a novel porous crystalline magnesium silicate having composition expressed according to Formulae I and II.

The alkylation is performed by combining the aromatic compound with an alkylation agent in the presence of the magnesium silicate catalyst. The reaction is conducted at elevated temperatures and pressures.

In the alkylation process, the aromatic compound to be alkylated is any aromatic compound containing at least one alkylatable ring position. Preferably, further substituents which would interfere with the alkylation by the formation of difficulty removable by-products or by deactivation of the catalyst whether due to tar formation or otherwise are absent. Most preferred aromatic compounds are benzene, toluene and ethylbenzene. One particularly unique advantage of the present process is the ability to alkylate a mixture of aromatic compounds, especially a mixture of benzene and toluene.

The alkylating agent is a convenient source of alkyl radicals. Suitable alkylating agents are α-olefins such as ethylene or propylene, lower ($C_1$—$C_7$) alkyl halids or mercaptans such as ethyl or propyl chlorides or mercaptans and lower ($C_1$—$C_7$) primary or secondary alcohols, especially methanol, ethanol or propanol. All alkylating agents previously known in the art may be employed in the present process. A preferred alkylating agent is ethylene.

Suitable temperatures are from 300°C to 600°C and preferably from 350°C to 500°C. Pressures from atmospheric to 500 psig (3447 kPa) may be employed. Preferred are pressures from 50 psig (345 kPa) to 350 psig (2413 kPa).

Optionally, modifying gases may be present in the reaction mixture. Suitably, hydrogen or an inert gas such as nitrogen, carbon dioxide or carbon monoxide may be present along with the reactants previously mentioned. The preferred modifying gas is hydrogen.

A further advantage of the present invention is that feed ratios of aromatic compound to alkylating agent may vary over wide extremes. On an equivalent basis, ratios from 20/1 to 1/1 are suitably employed. Preferred are ratios from 10/1 to 2/1.

The amount of catalyst employed is not critical to the success of the process since some amount of alkylated product is formed if suitable reaction times are provided. Advantageously, in a continuous process the reactants and catalyst are contacted in a weight hourly space velocity based on aromatic compound of from 0.1 to 50.

Where hydrogen is also present during the reaction, mole ratios of hydrogen/alkylating agent from 10:1 to 1:10 are suitably employed. Other diluent gases where employed may be present in about the same molar ratios.

An additional advantage of the present process is noted to be the almost total absence of disproportionation of alkylbenzenes when in contact with the present catalyst. For example, little or no formation of xylenes is observed when toluene is contacted with the catalyst under conditions of temperature and pressure suitably employed herein.

After prolonged use, particularly at extreme operating conditions, it may be desirable to regenerate the catalyst of the present process. The regeneration is performed in known manner, for example, by heating for several hours at a temperature of up to 600°C in the presence of steam, air or mixtures of air and nitrogen.

## Example 1

A solution *A* was made by combining 106 g of commercially available Philadelphia Quartz Sodium Silicate N® type (trademark of Philadelphia Quartz Company) (8.90 weight percent $Na_2O$, 28.7 weight percent $SiO_2$) with 132 g of $H_2O$. A second solution *B* was made by combining 180 g of $H_2O$, 40 g of NaCl, 26 g of $(C_3H_7)_4NBr$, 10.2 g $MgCl_2 \cdot 6H_2O$ and 8 g of concentrated $H_2SO_4$ (96 weight percent) to form a clear solution.

Solution *A* was transferred to a Waring® blender and the blender was started at the "high" setting. Solution *B* was added at once and the mixture was stirred vigorously for 1 minute. The resulting slurry was then placed inside a stainless steel autoclave, heated to 150°C under autogenous pressure and stirred. After 24 hours, the autoclave was cooled to room temperature and the solid product was isolated by filtration. The filter cake was washed several times with much water and then air dried at 110°C into a free flowing powder. X-ray powder diffraction (XRD) analysis of the powder gave a pattern similar to that reported for silicalite and ZSM-5.

## Example 2

A product made according to the procedure of Example 1 which is calcined overnight in air at 500°C to remove trapped organic matter produces changes in the relative intensities observed by XRD analysis.

Surface area measurements on a calcined solid, made according to the above procedure, by the single point BET method gave a measurement of 378 $m^2/g$.

Analysis of a product made according to the procedure in Example 1 by infrared spectroscopy using a Perkin-Elmer® Model 337 double-beam instrument produces a characteristic spectrum with two distinct bands in the 1200—980 $cm^{-1}$ region.

Differential thermal analysis of a solid silicate (as made according to the Procedure in Example 1) using a duPont® 990 thermal analysis unit equipped with a 1200°C furnace revealed a characteristic exotherm at 860°C. The sample was heated at a rate of 20°C/minute in a platinum crucible in an air atmosphere with an air flow rate of 50 $cm^3$/minute. The product was recovered after heating to 950°C and was analyzed by XRD and found to contain all the lines listed in Table III.

Example 3

The procedure of Example 1 was repeated but with a reaction temperature of 125°C rather than 150°C. The solid dried at 110°C was calcined at 500°C for 18 hours. Chemical analysis of the solid was done by neutron activation analysis and revealed on a molar ratio basis referred to $MgO:SiO_2$ (11.0), MgO (1.0), $Na_2O$ (0.38) and $Al_2O_3$ (0.015). The XRD analysis of the magnesium silicate product was consistent with the diffraction lines listed in Table III.

Example 4

A reaction mixture was prepared as follows: (a) 100 g of commercially available Ludox SM® (trademark of duPont) colloidal silica (30 weight percent $SiO_2$, 0.56 weight percent $Na_2O$) were mixed with 8.0 g of NaOH solution (50 weight percent NaOH) and 100 g of $H_2O$; (b) 26 g of $(C_3H_7)_4NBr$ were dissolved in 110 g of $H_2O$; (c) 10.2 g of $MgCl_2 \cdot 6H_2O$ were dissolved in 100 g of $H_2O$.

The mixtures of (a) and (b) were mixed in a high torque blender for a few seconds and then solution (c) was added maintaining the mixing for 1 minute to produce an apparently homogeneous mixture. The pH of the mixture was adjusted to 11 by addition of NaOH (50 weight percent NaOH). The pH adjusted mixture was then transferred to a stainless steel autoclave and stirred at 150°C for 30 hours under autogenous pressure. The product was recovered by filtration and rinsed with copious amounts of water. The lines observed by X-ray powder diffraction analysis of the solid match those values listed in Table III.

Example 5

A porous crystalline magnesium silicate that was prepared substantially according to the process of Examples 1—4 was calcined overnight to remove organic moieties. This calcined material was then slurried with hot 1N $NH_4NO_3$ overnight. The material recovered by filtering this slurry was dried for several hours at 110°C. Then one part of this magnesium silicate was mixed with one-half part kaolin clay and enough water to form a moist cake. The cake was dried and then calcined at 500°C in air for 5 hours. This material was then crushed into 6—12 mesh aggregates.

The crushed aggregate (9 g) was placed into the center portion of a ½" × 30" (1.2 cm × 76.2 cm) 316 stainless steel reactor tube with 8—12 mesh silica on both sides acting as a support and aid to the uniform heating of the catalyst. A ⅛" (0.32 cm) thermowell inside the reactor was equipped with a thermocouple for measuring reactor temperatures. The reactor was placed inside an electric furnace and heated to 370°C. A hydrogen gas feed was begun and the pressure increased to 100 psig (689.5 kPa) and then toluene was fed to the reactor. When toluene was detected downstream from the reactor, the ethylene feed was started.

The toluene was pumped into the reactor at a rate of 121 g per hour, hydrogen gas was added at a rate of 240 $cm^3$ per minute as measured at ambient pressure and temperature, and ethylene was added at a rate of 75 $cm^3$ per minute as measured at 21°C and 760 mm Hg pressure. The reactor was operated at 100 psig (689.5 kPa) with a negligible pressure drop across the catalyst bed.

About 3½ hours after the ethylene feed stream was turned on, a sample of the reactor effluent was taken and analyzed by conventional gas chromatographic methods. The results are given in Table IV below.

TABLE IV

Reactor Effluent Analysis

| | Mole Percentage in Liquid Effluent |
|---|---|
| Benzene | 0.03 |
| Toluene | 88.15 |
| EBX* | 0.126 |
| para-Ethyltoluene | 7.83 |
| ortho-Ethyltoluene | - |
| meta-Ethyltoluene | 3.55 |
| DEB** | 0.15 |

*Ethylbenzene and xylenes.

**Diethylbenzene.

The molar feed ratios for the above process were approximately 7:1:3 for toluene to ethylene to hydrogen. The above analysis gives approximately an 80.1 percent conversion rate to ethyltoluene for ethylene based upon a maximum theoretical conversion calculated by dividing the mole per hour feed rate for ethylene by that for toluene.

Example 6

The reaction conditions of Example 5 were substantially repeated employing varying temperatures, pressures and amounts of hydrogen, ethylene, aromatic compound and nitrogen identified in Table V. Except where noted, analysis of the reactor effluent by gas/liquid chromatograph was made after 24 hours of continuous operation at reaction conditions. The catalyst was 8.0 g of supported catalyst prepared according to the procedure of Example 5. In the table, ET is ethyltoluene, p-ET is para-ethyltoluene, m-ET is meta-ethyltoluene, and o-ET is ortho-ethyltoluene.

## TABLE V

| Run | °C | psig (pKa) | $H_2$ (m/hr) | $C_2H_4$ (m/hr) | toluene (m/hr) | $N_2$ (m/hr) | % $C_2H_4$ Conv. | % p-ET | % m-ET | % o-ET | % ET Sel. | Note |
|---|---|---|---|---|---|---|---|---|---|---|---|---|
| 1 | 380 | 100 (689.5) | 0.45 | 0.15 | 1.06 | 0 | 69 | 54.0 | 45.4 | 0.6 | 96 | a |
| 2 | 380 | 100 (689.5) | 0.45 | 0.07 | 0.46 | 0 | 78 | 44.6 | 54.7 | 0.7 | 97 | a |
| 3 | 380 | 200 (1378.9) | 0.53 | 0.07 | 0.46 | 0 | 89 | 41.3 | 57.9 | 0.8 | 96 | a |
| 4 | 460 | 35 (221.3) | 0.09 | 0.30 | 2.06 | 0 | 48 | 52.8 | 46.3 | 0.9 | 97 | a |
| 5 | 480 | 200 (1378.9) | 0.14 | 0.30 | 2.06 | 0 | 89 | 36.1 | 62.2 | 1.7 | 96 | a,b |
| 6 | 385 | 100 (689.5) | 0 | 0.15 | 1.08 | 0.06 | 74 | 51.2 | 48.0 | 0.8 | 94 | c |
| 7 | 408 | 280 (1930.5) | 0.45 | 0.25 | 1.05 | 0 | 79 | 43.0 | 55.8 | 1.2 | 95 | d |

(m/hr) = (mole/hr)

[a] Conversion was essentially constant during 24 hours.

[b] Air regeneration between runs 4 and 5.

[c] Air regeneration between runs 5 and 6, results after 2 hours operation.

[d] Air regeneration between runs 6 and 7, results after 4 hours operation.

Example 7

The reaction conditions of Example 5 were substantially repeated excepting that the aromatic reactant was benzene. Accordingly, 8.0 g of catalyst prepared substantially according to the methods of Example 5 was employed. The process conditions and results upon analysis of the reactor effluent after 18 hours of operation at 415°C and 100 psig (689.5 kPa) are contained in Table VI.

TABLE VI

| $H_2$ (mole/hr) | $C_2H_4$ (mole/hr) | Benzene (mole/hr) | % $C_2H_4$ Conv. | % EB Sel. |
|---|---|---|---|---|
| 0.55 | 0.42 | 1.27 | 90 | 70 |

Example 8

The reaction conditions of Example 5 were substantially repeated excepting that the alkylating agent employed was methanol. Accordingly, 20.0 g of bound catalyst pellets prepared substantially according to the method of Example 5 were employed. The results obtained by analysis of effluent after 24 hours of continuous operation are contained in Table VII. The reaction conditions are as follows: Temperature — 379°C, Pressure — 25 psig (172.4 kPa), feed rates: toluene — 1.02 mole/hr, methanol — 0.15 mole/hr, hydrogen — 0.16 mole/hr.

TABLE VII

| % Methanol Conversion | % Xylene Selectivity | Xylene distribution | | |
|---|---|---|---|---|
| | | ortho- | meta- | para- |
| 79 | 73 | 29.4 | 35.2 | 35.4 |

Example 9

The reaction conditions of Example 5 were again substantially repeated excepting that the aromatic compound was a mixture of benzene and toluene. Accordingly, 8.0 g of magnesium silicate catalyst prepared substantially according to the procedure of Example 5 were employed. The following conditions were employed: reactor temperature — 422°C, pressure — 100 psig (689.5 kPa), $C_2H_4$ feed rate — 170 $cm^3$/min measured at ambient temperature and pressure, $H_2$ feed rate — 78 $cm^3$/min measured at ambient temperature and pressure. An aromatic feed of benzene and toluene in a molar ratio of 89:11 was supplied to the reactor at a rate of 99.2 g/hr.

After 2 hours continuous operation at the above reaction conditions, the reactor effluent was analyzed by gas-liquid chromatography. Results are contained in Table VIII.

TABLE VIII

| Component | Mole % |
|---|---|
| benzene | 64.03 |
| toluene | 8.92 |
| ethylbenzene | 19.36 |
| p-ethyltoluene | 1.44 |
| m-ethyltoluene | 1.94 |
| o-ethyltoluene | 0.07 |
| p-diethylbenzene | 1.92 |
| m-diethylbenzene | 2.18 |
| o-diethylbenzene | 0.03 |

Benzene conversion was 27.9 percent. Toluene conversion was 20.0 percent. Ethylene conversion was 91.6 percent. The process is seen to be particularly advantageous in that xylene formation due to

disproportionation of toluene is extremely low. Because ethylbenzene and xylenes are extremely difficult to separate by fractional distillation, the present process for alkylating a mixture of benzene and toluene is highly advantageous due to the near lack of xylene formation.

Example 10

A reaction mixture was prepared as follows: (a) 530 g of Philadelphia Quartz N® type sodium silicate were mixed with 660 g $H_2O$, and (b) 200 g of NaCl, 50.8 g $MgCl_2 \cdot 6H_2O$, 130 g of $(C_3H_7)_4NBr$, 40 g $H_2SO_4$ solution (96 weight percent $H_2SO_4$) and 900 g of $H_2O$ were mixed.

Solution (a) was transferred to a high torque blender and solution (b) was added to it maintaining continuous stirring. When the addition was finished the slurry was stirred for 1 minute. The homogeneous slurry was transferred to a stainless steel autoclave and stirred at 125°C under autogeneous pressure for 26 hours. The resulting product was separated by filtration and dried at 110°C overnight.

Analysis of the product by X-ray powder diffraction shows that the magnesium silicate contains the lines listed in Table III. Surface area analysis of the calcined product by the one point BET method gave 395 $m^2/g$.

A portion of the product dried at 110°C was extracted with EDTA, calcined at 500°C and separate samples of this portion were ion-exchanged with 0.01 M NaOH and 0.01 M KOH solutions, respectively. Results of analysis of the exchanged products by neutron activation analysis are contained in Table IX. Results are on a molar basis referred to $M_gO$.

TABLE IX

| Solid | $SiO_2$ | MgO | $Na_2O$ | $K_2O$ | $Al_2O_3$ |
|---|---|---|---|---|---|
| NaOH Xged. | 8.9 | 1.0 | 0.040 | 0.005 | 0.01 |
| KOH Xged. | 8.3 | 1.0 | 0.005 | 0.040 | 0.01 |

Infrared analysis of the solid either as made, or after calcination, or after NaOH or KOH ion-exchange reveals two characteristic bands in the 1200—980.0 $cm^{-1}$ region. From the above data it is seen that the ion-exchange capacity is greater than that attributable to the presence of aluminum.

Example 11

This synthesis was carried out according to the following procedure: (a) 600 g of Ludox SM® colloidal silica (30 weight percent $SiO_2$, 0.56 weight percent $Na_2O$), 63.5 g of KOH pellets and 650 g of $H_2O$ were mixed; (b) 305 g of KCl, 156 g of $(C_3H_7)_4NBr$ and 660 g of $H_2O$ were mixed; and (c) 61 g of $MgCl_2 \cdot 6H_2O$ were dissolved in 600 g of $H_2O$.

Solution (b) was added to solution (a) in a high torque blender followed by solution (c). The final mixture was stirred for 1 minute into a homogeneous slurry. The slurry was transferred to a stainless steel autoclave and stirred at 175°C for approximately 72 hours. The product was recovered by filtration, washed with $H_2O$ and dried at 110°C overnight in air.

XRD analysis of the solid indicates that the material is crystalline corresponding to the features listed in Table III. Infrared analysis of the solid shows two characteristic bands in the 1200—980 $cm^{-1}$ region.

A portion of the solid was extracted with EDTA, etc., in the manner indicated in Example 10 and analyzed by neutron activation analysis. The results on a molar basis referred to MgO correspond to the NaOH and KOH exchanged samples. The results are contained in Table X.

TABLE X

| Solid | $SiO_2$ | MgO | $Na_2O$ | $K_2O$ | $Al_2O_3$ |
|---|---|---|---|---|---|
| NaOH Xged. | 8.5 | 1.0 | 0.040 | 0.02 | 0.008 |
| KOH Xged. | 8.0 | 1.0 | 0.002 | 0.05 | 0.009 |

Differential thermal analysis, according to the procedure of Example 2, of the solid as made gives a characteristic exotherm at 880°C.

Example 12

A solution A was made combining 795 g of N® type sodium silicate (trademark of the PQ Corporation) (8.90 percent $Na_2O$, 28.7 percent $SiO_2$) with 1020 g of $H_2O$. A second solution B was made combining 300 g of NaCl, 76.2 g of $MgCl_2 \cdot 6H_2O$, 195 g of $(C_3H_7)_4NBr$, 1230 g of $H_2O$ and 55 g of concentrated $H_2SO_4$ (96 percent) to form a clear solution.

The solutions were mixed pumping 1815 g of solution A and 1754 g of solution B at 250 ml/min each into the mixing head of a Tekmar mixer model LD-45. The mixer was set at 90 percent of its maximum speed. A smooth slurry was obtained. This slurry (2432 g) was transferred into a Teflon lined autoclave at 125°C under autogeneous pressure and stirred at 500 rpm. After 70 hours, the autoclave was cooled to room temperature and the solid product was isolated by filtration.

About one gram of the filter cake was reslurried in 15 ml of $H_2O$ in a test tube and the mixture was centrifugated to separate the solids from the liquid. This operation was repeated twice discarding the clear filtrate. Two layers of solid were observed in the test tube. The two layers were separated and analyzed. The top layer is amorphous and the lower one is crystalline as shown by X-ray analysis. The crystalline layer has all the diffraction peaks listed in Table III.

Energy Dispersive Spectroscopy analysis shows Si, Na, and Mg in both layers. The amorphous one also contains Cl and higher concentrations of Na and Mg than the crystalline layer.

The remainder of the filter cake (1460 g) was used to separate the amorphous from the crystalline material following the same technique described above.

A high purity crystalline sample was made repeating the procedure 10 times. This sample was used for a variety of analysis.

Neutron activation analysis was used for Na, Mg, Al and Si, and C, H and N were determined by combustion. The composition is summarized in Table XI.

TABLE XI

Elemental analysis and molar ratios of purified NaMg silicate crystals

| Components | Weight Percent | Analytical Method |
|---|---|---|
| $SiO_2$ | 88.6 | Neutron Activation |
| MgO | 0.45 | Neutron Activation |
| $Na_2O$ | 1.22 | Neutron Activation |
| $Al_2O_3$ | 0.013 | Neutron Activation |
| C | 7.95 | Combustion |
| H | 1.86 | Combustion |
| N | 0.74 | Combustion |
| Total | 100.8 | |

| Elements | Molar Ratio |
|---|---|
| Si/Mg | 128.0 |
| Si/Al | 5500.0 |
| Na/Mg | 3.56 |
| Si/N | 27.0 |
| C/N | 12.5 |

Electron microprobe analysis of thin sections of the crystalline particles indicates that the magnesium concentration in the aggregates is not uniform, ranging from 400 ppm at the core to 3000 ppm at the surface. Aluminium was not detected within the crystalline particles. The amorphous material present in the samples not purified contains 13 percent magnesium as a silicate mixed with NaCl crystals.

Microscopic analysis of the purified crystalline materials shows uniform particles of 8 microns in diameter with rounded corners and almost square shape.

X-ray analysis of the purified crystals using a Gunier camera indicates that the symmetry is

orthorhombic. The unit cell dimensions are: a(Å)=20.024(5), b(A)=13.380(4), c(Å)=19.903(3), β(degrees)=90. The unit cell volume V($Å^3$)=5.332(2). The X-ray powder diffraction pattern of the sample experiences a variety of changes when the crystalline material is calcined to remove organic moieties. First, the reflection at about 12.05 degrees 2θ drops in intensity and splits when the solid is calcined. The doublet at around 14.8 degrees 2θ collapses into a single peak and its intensity decreases. The single peak at 24.5 degrees splits into two peaks of lower intensity. A similar change occurs at the peak at 26.1 degrees 2θ, etc. Also, there are obvious changes in intensity of the peaks between 7—10 degrees and 22—25 degrees 2θ. These phenomena are associated with a symmetry change from orthorhombic to monoclinic when the crystalline material is calcined.

BET adsorption-desorption isotherms of $N_2$ on the purified crystals after calcination in air at 500°C show a very flat and almost hysterisis free isotherm. The BET surface area is 521.07 $m^2/g$. The micropore volume (t-plot) is 0.191 $cm^3/g$. The least squares surface are (t-plot) is 1.95 $m^2/g$. Apparently, the calcined crystalline material is free of meso and macropores.

Infrared analysis of the crystalline material before and after calcination shows the presence of at least two distinct bands in the 1200—980 $cm^{-1}$ region and at least one or more additional bands at 1225±10 $cm^{-1}$, 800±20 $cm^{-1}$, 620±10 $cm^{-1}$, 550±20 $cm^{-1}$ and 450±20 $cm^{-1}$.

In addition to the obvious exotherms associated with combustion of organic moieties below 500°C, differential thermal analysis of the purified crystalline material shows an exotherm between 800°C and 900°C and another at 1020°C. This last exotherm (1020°C) is associated with a collapse of the crystalline material into amorphous material and/or cristobalite, a dense silica polymorph.

A calcined sample of the purified crystals was exchanged with 0.1 NaOH using 100 ml/g of solid equilibrating for one day at room temperature. Back exchange with 0.1 N KCl under similar conditions gave a capacity of 0.34 meq Na/g of solid.

The purified crystalline material was evaluated as an alkylation catalyst in the alkylation of toluene with ethylene and was found to be active with results comparable to those reported in Table V. These results demonstrate that separation of the amorphous and crystalline components is not essential to obtain an active catalyst.

## Claims

1. A porous crystalline magnesium silicate having a composition according to the following formula in terms of the molar ratios of oxide on a dry basis:

$$(M_{2/n}O)p(MgO)_x(R_2O_3)_y(SiO_2)_z \quad \text{(Formula I)}$$

wherein M is at least one cation having a valence n; R is a trivalent element or mixture of two or more thereof; p is from 0.1 to 20, x is from 0.1 to 12; y is from 0 to 3, and z is from 84 to 96, and $p/n > y$, further characterized by (1) an infrared spectrum exhibiting at least two peaks in the 1200—980 $cm^{-1}$ region, (2) non-ion-exchangeable magnesium contained in lattice, and (3) an X-ray diffraction trace having at least interplanar d spacings of 11.2 ± 0.2, 10.1 ± 0.2, 10.0 ± 0.2, 9.8 ± 0.2, 6.0 ± 0.2, 5.8 ± 0.2, 5.6 ± 0.2, 4.26 ± 0.1, 3.85 ± 0.05, 3.81 ± 0.05, 3.74 ± 0.03, 3.72 ± 0.03, and 3.64 ± 0.03.

2. A porous crystalline magnesium silicate as claimed in Claim 1, wherein p is from about 0.1 to 4; x is from 0.01 to 2; and z is from 90 to 96.

3. A porous crystalline magnesium silicate as claimed in Claim 2 wherein y is from zero to 1.0.

4. A porous crystalline magnesium silicate as claimed in any one of the preceding claims, wherein M is an alkali metal cation or hydrogen.

5. A porous crystalline magnesium silicate as claimed in any one of the preceding claims wherein R is at least one of chromium, iron, aluminum, boron or a mixture thereof.

6. A porous crystalline magnesium silicate as claimed in any one of the preceding claims, which has been acid-exchanged.

7. A porous crystalline magnesium silicate as claimed in any one of the preceding claims which also exhibits at least one additional infrared band at 1225 ± 10 $cm^{-1}$, 800 ± 20 $cm^{-1}$, 620 ± 10 $cm^{-1}$, 550 ± 20 $cm^{-1}$ and/or 450 ± 20 $cm^{-1}$.

8. A porous crystalline magnesium silicate as claimed in any one of the preceding claims, wherein said silicate, when subjected to differential thermal analysis using alumina as a reference and a heating rate of 20°C per minute in an air atmosphere at an air flow rate of 50 $cm^3$ per minute reveals a distinct exotherm at 870 ± 30°C.

9. A porous crystalline magnesium silicate as claimed in any one of the preceding claims, wherein y is zero.

10. An alkylation catalyst comprising a porous crystalline magnesium silicate as claimed in any one of the preceding claims.

11. A process for preparing a porous crystalline magnesium silicate corresponding to the following formula in terms of the molar ratios of oxide on a dry basis:

$$(M_{2/n}O)p(MgO)_x(R_2O_3)_y(SiO_2)_z \quad \text{(Formula I)}$$

wherein M is at least one cation having a valence n; R is a trivalent element or mixture thereof; p is from 0.1 to 20, x is from 0.1 to 12, y is from 0 to 3, and 2 is from 84 to 96, further characterized by (1) an infrared spectrum exhibiting at least two peaks in the 1200—980 $cm^{-1}$ region, (2) non-ion-exchangeable magnesium contained in lattice, and (3) an X-ray diffraction trace having at least interplanar d spacings of 11.2 ± 0.2, 10.1 ± 0.2, 10.0 ± 0.2, 9.8 ± 0.2, 6.0 ± 0.2, 5.8 ± 0.2, 5.6 ± 0.2, 4.26 ± 0.1, 3.85 ± 0.05, 3.81 ± 0.05, 3.74 ± 0.03, 3.72 ± 0.03, and 3.64 ± 0.03, which process comprises

(a) combining a silicon source, which is colloidal silica, sodium silicate, organosilicate, amorphous silica or silica gel; a magnesium source which is a water-soluble magnesium salt, a magnesium-containing complex ion or a slightly soluble magnesium compound; water; a chloride, sulfate or hydroxide of sodium, potassium, rubidium or caesium; and a crystallization promoter which is a quaternary ammonium ion salt or hexamethylene diamine to form a reaction mixture;

(b) vigorously mixing the reaction mixture for a sufficient time to produce a homogeneous appearing gel; and

(c) allowing said gel to form crystals.

12. A process as claimed in Claim 11, further characterized by adjusting the pH of the said reaction mixture to pH 11 by addition of acid or base.

13. A process as claimed in Claim 12, further characterized by supplying heat to said crystallization step (c) at a pressure sufficient to prevent said reaction mixture or components thereof from vaporizing at such a rapid rate as to interfere with the crystallization.

14. A process as claimed in Claim 13, wherein said heating during crystallization is such as to maintain the gel at a temperature of from 80°C to 200°C.

15. A process as claimed in any one of Claims 11 to 14, wherein said crystallization promoter is a quaternary ammonium ion; said silicon source is colloidal silica or sodium silicate; said magnesium source is a water-soluble magnesium salt; and said alkali metal source is a salt or hydroxide of sodium or potassium.

16. A process as claimed in Claim 15, wherein said magnesium salt is magnesium chloride; said quaternary ammonium ion is tetrapropyl ammonium bromide and said heating during crystallization step is such as to maintain the gel at a temperature of from 125°C to 150°C.

17. A process for alkylating an aromatic hydrocarbon characterized by contacting an alkylating agent with an aromatic hydrocarbon containing at least one alkylatable ring position under reactive conditions in the presence of a catalytic amount of a porous crystalline magnesium silicate as claimed in any one of Claims 1 to 9.

18. A process according to Claim 17, wherein the aromatic hydrocarbon is toluene, benzene, ethylbenzene or a mixture of two or more thereof.

19. A process according to Claim 17 or Claim 18, wherein said alkylating agent is an α-olefin, lower ($C_1$—$C_7$) alkyl halide, lower ($C_1$—$C_7$) alkyl mercaptan, lower ($C_1$—$C_7$) primary alcohol or a lower ($C_1$—$C_7$) secondary alcohol; the ratio of said aromatic hydrocarbon to said alkylating agent is from 20:1 to 1:1 and the reaction is conducted at a temperature of from 300°C to 600°C and pressure of from atmospheric to 500 psig (3447 kPa).

20. A process according to Claim 19, wherein the alkylating agent is ethylene, propylene, ethyl chloride, propyl chloride, ethyl mercaptan, propyl mercaptan, methanol, ethanol or propanol.

21. A process according to Claim 19 or Claim 20, wherein the ratio of aromatic hydrocarbon to alkylating agent is from 10:1 to 2:1.

22. A process according to any one of Claims 19 to 21, wherein the temperature is from 350°C to 500°C.

23. A process according to any one of Claims 19 to 22, wherein the pressure is from 50 psig (345 kPa) to 350 psig (2413 kPa).

24. A process according to any one of Claims 17 to 23, wherein hydrogen gas is additionally present in the reaction mixture.

25. A continuous process according to any one of Claims 17 to 24, wherein the reactants and catalyst are contacted in a weight hourly space velocity based on aromatic compound of from 0.1 to 50.

26. A porous crystalline magnesium situated obtainable by a process as claimed in any one of Claims 11 to 16.

**Patentansprüche**

1. Poröses kristallines Magnesiumsilikat mit einer Zusammensetzung gemäß folgender Formel bezüglich der Molverhältnisse an Oxid auf Trockenbasis:

$$(M_{2/n}O)p(MgO)_x(R_2O_3)_y(SiO_2)_z \qquad \text{(Formel I)}$$

worin M mindestens ein Kation mit einer Valenz n ist, R ein trivalentes Element oder ein Gemisch aus zwei oder mehreren davon ist, p von 0,1 bis 20 ist, x von 0,1 bis 12 ist, y von 0 bis 3 ist, z von 84 bis 96 ist und p/n > y ist, weiterhin gekennzeichnet durch (1) ein Infrarotspektrum, das mindestens zwei Peaks in der Region von 1200 bis 980 $cm^{-1}$ besitzt, (2) im Gitter enthaltenes, nicht Ionen-austauschbares Magnesium und (3) ein Röntgendiffraktionsbild, das mindestens interplanare d-Abstände von 11,2 ± 0,2, 10,1 ± 0,2, 10,0 ± 0,2, 9,8

$\pm$ 0,2, 6,0 $\pm$ 0,2, 5,8 $\pm$ 0,2, 5,6 $\pm$ 0,2, 4,26 $\pm$ 0,1, 3,85 $\pm$ 0,05, 3,81 $\pm$ 0,05, 3,74 $\pm$ 0,03, 3,72 $\pm$ 0,03 und 3,64 $\pm$ 0,03 besitzt.

2. Poröses kristallines Magnesiumsilikat nach Anspruch 1, worin p von ungefähr 0,1 bis 4 ist, x von 0,01 bis 2 ist und z von 90 bis 96 ist.

3. Poröses kristallines Magnesiumsilikat nach Anspruch 2, worin y von 0 bis 1,0 ist.

4. Poröses kristallines Magnesiumsilikat nach einem der vorhergehenden Ansprüche, worin M ein Alkalimetallkation oder Wasserstoff ist.

5. Poröses kristallines Magnesiumsilikat nach einem der vorhergehenden Ansprüche, worin R mindestens aus einem Stoff von Chrom, Eisen, Aluminium, Bor oder einem Gemisch davon besteht.

6. Poröses kristallines Magnesiumsilikat nach einem der vorhergehenden Ansprüche, des sauer ausgetauscht wurde.

7. Poröses kristallines Magnesiumsilikat nach einem der vorhergehenden Ansprüche, das außerdem mindestens eine zusätzlich Infrarotbande bei 1225 $\pm$ 10 $cm^{-1}$, 800 $\pm$ 20 $cm^{-1}$, 620 $\pm$ 10 $cm^{-1}$, 550 $\pm$ 20 $cm^{-1}$, und/oder 450 $\pm$ 20 $cm^{-1}$ besitzt.

8. Poröses kristallines Magnesiumsilikat nach einem der vorhergehenden Ansprüche, worin das Silikat bei einer Differential-Thermoanalyse unter Verwendung von Aluminiumoxid als Referenzsubstanz und einer Heizgeschwindigkeit von 20°C pro Minute an einer Luftatmosphäre bei einer Luftflußgeschwindigkeit von 50 $cm^3$ pro Minute eine charakteristische Exotherme bei 870 $\pm$ 30°C aufweist.

9. Poröses kristallines Magnesiumsilikat nach einem der vorhergehenden Ansprüche, worin y 0 ist.

10. Alkylierungs-Katalysator, der ein poröses kristallines Magnesiumsilikat nach einem der vorhergehenden Ansprüche enthält.

11. Verfahren zur Herstellung eines porösen kristallinen Magnesiumsilikats gemäß folgender Formel, bezogen auf die Molverhältnisse an Oxid auf Trockenbasis:

$$(M_{2/n}O)p(MgO)_x(R_2O_3)_y(SiO_2)_z \qquad \text{(Formel I)}$$

worin M mindestens ein Kation mit einer Valenz n ist, R ein trivalentes Element oder ein Gemisch davon ist, p von 0,1 bis 20 ist, x von 0,1 bis 12 ist, y von 0 bis 3 ist und z von 84 bis 96 ist, weiterhin gekennzeichnet durch (1) ein Infrarotspektrum, das mindestens zwei Peaks im Bereich von 1200 bis 980 $cm^{-1}$ aufweist, (2) im Gitter enthaltenes, nicht Ionen-austauschbares Magnesium und (3) ein Röntgendiffraktionsmuster, das mindestens interplanare d-Abstände von 11,2 $\pm$ 0,2, 10,1 $\pm$ 0,2, 10,0 $\pm$ 0,2, 9,8 $\pm$ 0,2, 6,0 $\pm$ 0,2, 5,8 $\pm$ 0,2, 5,6 $\pm$ 0,2, 4,26 $\pm$ 0,1, 3,85 $\pm$ 0,05, 3,81 $\pm$ 0,05, 3,74 $\pm$ 0,03, 3,72 $\pm$ 0,03 und 3,64 $\pm$ 0,03 besitzt, wobei das Verfahren umfaßt:

(a) das Vereinigen eines Silizium-Ausgangsstoffes, bei dem es sich um kolloidales Siliziumoxid, Natriumsilikat, Organosilikat, amorphes Siliziumoxid oder Kieselgel handelt, eines Magnesium-Ausgangsstoffes, bei dem es sich um ein wasserlösliches Magnesiumsalz, ein Magnesium enthaltendes Komplexion oder eine schwach lösliche Magnesiumverbindung handelt, Wasser, eines Chlorides, Sulfats oder Hydroxids von Natrium, Kalium, Rubidium oder Cäsium und eines Kristallisations-Aktivators, bei dem es sich um ein quaternäres Ammoniumionensalz oder Hexamethylendiamin handelt, so daß ein Reaktionsgemisch entsteht,

(b) ein starkes Vermischen des Reaktionsgemisches über einen Zeitraum, der ausreichend ist, um ein homogen erscheinendes Gel zu erzeugen und

(c) zu ermöglichen, daß das Gel Kristalle bildet.

12. Verfahren nach Anspruch 11, weiterhin gekennzeichnet durch Einstellen des pH-Werts des Reaktionsgemisches auf pH 11 durch Zugabe von Säure oder Base.

13. Verfahren nach Anspruch 12, weiterhin gekennzeichnet durch Wärmezufuhr beim Kristallisationsschritt (c) bei ausreichendem Druck, um ein Verdampfen des Reaktionsgemisches oder Teilen davon mit einer so hohen Geschwindigkeit zu verhindern, welche die Kristallisation beeinträchtigen würde.

14. Verfahren nach Anspruch 13, worin das Erhitzen während der Kristallisation so geschieht, daß das Gel bei einer Temperatur von 80°C bis 200°C gehalten wird.

15. Verfahren nach einem der Ansprüche 11 bis 14, worin der Kristallisations-Aktivator ein quaternäres Ammonium ist, der Silizium-Ausgangsstoff kolloidales Siliziumoxid oder Natriumsilikat ist, der Magnesium-Ausgangsstoff ein wasserlösliches Magnesiumsalz ist und der Alkalimetall-Ausgangsstoff ein Salz oder Hydroxid von Natrium oder Kalium ist.

16. Verfahren nach Anspruch 15, worin das Magnesiumsalz Magnesiumchlorid ist, das quaternäre Ammoniumion Tetrapropyl-Ammoniumbromid ist und das Erhitzen während des Kristallisationsschritts so geschieht, daß das Gel bei einer Temperatur von 125°C bis 150°C gehalten wird.

17. Verfahren zur Alkylierung eines aromatischen Kohlenwasserstoffs, dadurch gekennzeichnet, daß man ein Alkylierungsmittel mit einem aromatischen Kohlenwasserstoff, der mindestens eine alkylierbare Ringposition besitzt, unter Reaktionsbedingungen in Gegenwart einer katalytischen Menge eines porösen kristallinen Magnesiumsilikats nach einem der Ansprüche 1 bis 9 in Kontakt bringt.

18. Verfahren nach Anspruch 17, worin der aromatische Kohlenwasserstoff Toluol, Benzol, Ethylbenzol oder ein Gemisch aus zwei oder mehreren dieser Substanzen ist.

19. Verfahren nach Anspruch 17 oder 18, worin das Alkylierungsmittel ein α-Olefin, ein niederes

$(C_1—C_7)$-Alkylhalogenid, ein niederes $(C_1—C_7)$-Alkylmercaptan, ein niederer $(C_1—C_7)$ primärer Alkohol oder ein niederer $(C_1—C_7)$ sekundärer Alkohol ist, das Verhältnis von aromatischem Kohlenwasserstoff zu Alkylierungsmittel von 20:1 bis 1:1 ist und die Reaktion bei einer Temperatur von 300°C bis 600°C und einem Druck von atmosphärischem Druck bis 500 psig (3447 kPa) durchgeführt wird.

20. Verfahren nach Anspruch 19, worin das Alkylierungsmittel Ethylen, Propylen, Ethylchlorid, Propylchlorid, Ethylmercaptan, Propylmercaptan, Methanol, Ethanol oder Propanol ist.

21. Verfahren nach Anspruch 19 oder 20, worin das Verhältnis von aromatischen Kohlenwasserstoff zu Alkylierungsmittel von 10:1 bis 2:1 ist.

22. Verfahren nach einem der Ansprüche 19 bis 21, worin die Temperatur von 350°C bis 500°C ist.

23. Verfahren nach einem der Ansprüche 19 bis 22, worin der Druck von 50 psig (345 kPa) bis 350 psig (2413 kPa) ist.

24. Verfahren nach einem der Ansprüche 17 bis 23, worin zusätzlich Wasserstoffgas im Reaktionsgemisch vorhanden ist.

25. Kontinuierliches Verfahren nach einem der Ansprüche 17 bis 24, worin die Reaktanden und der Katalysator in einer stündlichen Raumgewichtsrate von 0,1 bis 50, bezogen auf die aromatische Verbindung in Kontakt gebracht werden.

26. Poröses kristallines Magnesiumsilikat, erhältlich durch ein Verfahren nach einem der Ansprüche 11 bis 16.

**Revendications**

1. Silicate de magnésium cristallin poreux, ayant une composition selon la formule suivante, exprimée en rapports molaires d'oxydes a l'état anhydre:

$$(M_{2/n}O)_p(MgO)_x(R_2O_3)_y(SiO_2)_z \quad \text{(formule I)}$$

dans laquelle M est au moins un cation de valence n; R est un élément trivalent ou un mélange de deux éléments de ce type ou plus; p vaut de 0,1 à 20; x vaut de 0,1 à 12; y vaut de 0 à 3 et z vaut de 84 à 96; et p/n > y, caractérisé en outre par (1) un spectre infrarouge présentant au moins deux pics dans la région de 1200—980 $cm^{-1}$, (2) du magnésium non échangeable avec des ions, contenu dans le réseau cristallin et (3) un diagramme de diffraction des rayons X indiquant au moins des distances réticulaires de 11,2 ± 0,2, 10,1 ± 0,2, 10,0 ± 0,2, 9,8 ± 0,2, 6,0 ± 0,2, 5,8 ± 0,2, 5,6 ± 0,2, 4,26 ± 0,1, 3,85 ± 0,05, 3,81 ± 0,05, 3,74 ± 0,03, 3,72 ± 0,03 et 3,64 ± 0,03.

2. Silicate de magnésium cristallin poreux selon la revendication 1, dans lequel p vaut d'environ 0,1 à 4, x vaut de 0,01 à 2 et z vaut de 90 à 96.

3. Silicate de magnésium cristallin poreux selon la revendication 2, dans lequel y vaut de zéro à 1,0.

4. Silicate de magnésium cristallin poreux selon l'une quelconque des revendications précédentes, dans lequel M est un cation métal alcalin ou l'hydrogène.

5. Silicate de magnésium cristallin poreux selon l'une quelconque des revendications précédentes, dans lequel R est au moins un élément parmi le chrome, le fer, l'aluminium, le bore ou un mélange de ceux-ci.

6. Silicate de magnésium cristallin poreux selon l'une quelconque des revendications précédentes, qui a subi un échange avec un acide.

7. Silicate de magnésium cristallin poreux selon l'une quelconque des revendications précédentes, qui en outre présente au moins un bande infrarouge supplémentaire à 1225 ± 10 $cm^{-1}$, 800 ± 20 $cm^{-1}$, 620 ± 10 $cm^{-1}$, 550 ± 20 $cm^{-1}$ et/ou 450 ± 20 $cm^{-1}$.

8. Silicate de magnésium cristallin poreux selon l'une quelconque des revendications précédentes, qui, soumis à une analyse thermique différentielle avec utilisation d'alumine en tant que substance de référence et une vitesse de chauffage de 20°C par minute dans une atmosphère d'air, à un débit d'air de 50 $cm^3$ par minute, présente un pic exothermique net à 870 ± 30°C.

9. Silicate de magnésium cristallin poreux selon l'une quelconque des revendications précédentes, dans lequel y vaut zéro.

10. Catalyseur d'alkylation comprenant un silicate de magnésium cristallin poreux selon l'une quelconque des revendications précédentes.

11. Procédé pour la préparation d'un silicate de magnésium cristallin poreux, correspondant à la formule suivante, exprimée en rapports molaires d'oxydes à l'état anhydre:

$$(M_{2/n}O)_p(MgO)_x(R_2O_3)_y(SiO_2)_z \quad \text{(formule I)}$$

dans laquelle M est au moins un cation de valence n; R est un élément trivalent ou un mélange d'éléments de ce type; p vaut de 0,1 à 20; x vaut de 0,1 à 12; y vaut de 0 à 3 et z vaut de 84 à 96; caractérisé en outre par (1) un spectre infrarouge présentant au moins deux pics dans la région de 1200—980 $cm^{-1}$, (2) du magnésium non échangeable avec des ions, contenu dans le réseau cristallin et (3) un diagramme de diffraction des rayons X indiquant au moins des distances réticulaires de 11,2 ± 0,2, 10,1 ± 0,2, 10,0 ± 0,2, 9,8 ± 0,2, 6,0 ± 0,2, 5,8 ± 0,2, 5,6 ± 0,2, 4,26 ± 0,1, 3,85 ± 0,05, 3,81 ± 0,05, 3,74 ± 0,03, 3,72 ± 0,03 et 3,64 ±

0,03, lequel procédé comprend

(a) la réunion d'une source de silicium, laquelle est de la silice colloïdale, du silicate de sodium, un organosilicate, de la silice amorphe ou du gel de silice; une source de magnésium, laquelle est un sel de magnésium soluble dans l'eau, un ion complexe contenant du magnésium ou un composé de magnésium légèrement soluble; de l'eau; un chlorure, sulfate ou hydroxyde de sodium, potassium, rubidium ou césium; et un agent favorisant la cristallisation, qui est un sel d'ammonium quaternaire ou l'hexaméthylénediamine, pour former un mélange réactionnel;

(b) le mélangeage énergique du mélange réactionnel pendant une durée suffisante pour l'obtention d'un gel d'aspect homogène; et

(c) l'abandon du gel au repos pour la formation de cristaux.

12. Procédé selon la revendication 11, caractérisé en outre par l'adjustment du pH dudit mélange réactionnel à pH 11 par addition d'un acide ou d'une base.

13. Procédé selon la revendication 12, caractérisé en outre par l'application de chaleur dans ladite étape de cristallisation (c), sous une pression suffisante pour empêcher la vaporisation dudit mélange réactionnel ou de composants de celui-ci à une vitesse de nature à gêner la cristallisation.

14. Procédé selon la revendication 13, dans lequel ledit chauffage pendant la cristallisation est tel que le gel est maintenu à une température de 80 à 200°C.

15. Procédé selon l'une quelconque des revendications 11 à 14, dans lequel l'agent favorisant la cristallisation est un ion ammonium quaternaire; ladite source de silicium est de la silice colloïdale ou du silicate de sodium; ladite source de magnésium est un sel de magnésium soluble dans l'eau; et ladite source de métal alcalin est un sel ou hydroxyde de sodium ou de potassium.

16. Procédé selon la revendication 15, dans lequel le sel de magnésium est le chlorure de magnésium; ledit ion ammonium quaternaire est le bromure de tétrapropylammonium et ledit chauffage pendant l'étape de cristallisation est tel que le gel est maintenu à une température de 125 à 150°C.

17. Procédé pour l'alkylation d'un hydrocarbure aromatique, caractérisé par la mise en contact d'un agent d'alkylation avec un hydrocarbure aromatique contenant au moins une position alkylable sur le noyau dans les conditions réactionnelles, en présence d'une quantité catalytique d'un silicate de magnésium cristallin poreux tel que défini dans l'une quelconque des revendications 1 à 9.

18. Procédé selon la revendication 17, dans lequel l'hydrocarbure aromatique est le toluène, le benzène, l'éthylbenzène ou un mélange de deux de ceux-ci ou plus.

19. Procédé selon la revendication 17 ou la revendication 18, dans lequel l'agent d'alkylation est une α-olefine, un halogénure d'alkyle inférieur en $C_1$—$C_7$, un alkylmercaptan inférieur en ($C_1$—$C_7$), un alcool primaire inférieur en $C_1$—$C_7$ ou un alcool secondaire inférieur en $C_1$—$C_7$; le rapport dudit hydrocarbure aromatique audit agent d'alkylation vaut de 20:1 à 1:1, et la réaction est effectuée à une température de 300 à 600°C et sous une pression dans la plage de la pression atmosphérique à une pression manométrique de 3 447 kPa (500 psi).

20. Procédé selon la revendication 19, dans lequel l'agent d'alkylation est l'éthylène, le propylène, le chlorure d'éthyle, le chlorure de propyle, l'éthylmercaptan, le propylmercaptan, le méthanol, l'éthanol ou le propanol.

21. Procédé selon la revendication 19 ou 20, dans lequel le rapport de l'hydrocarbure aromatique à l'agent d'alkylation vaut de 10:1 à 2:1.

22. Procédé selon l'une quelconque des revendications 19 à 21, dans lequel la température est de 350 à 500°C.

23. Procédé selon l'une quelconque des revendications 19 à 22, dans lequel la pression manométrique est de 345 kPa (50 psi) à 2 413 kPa (350 psi).

24. Procédé selon l'une quelconque des revendications 17 à 23, dans lequel de l'hydrogène gazeux est en outre présent dans le mélange réactionnel.

25. Procédé en continu selon l'une quelconque des revendications 17 à 24, dans lequel les corps en réaction et le catalyseur sont mis en contact à une vitesse spatiale pondérale horaire de 0,1 à 50, par rapport au composé aromatique.

26. Silicate de magnésium cristallin poreux préparable par le procédé selon l'une quelconque des revendications 11 à 16.

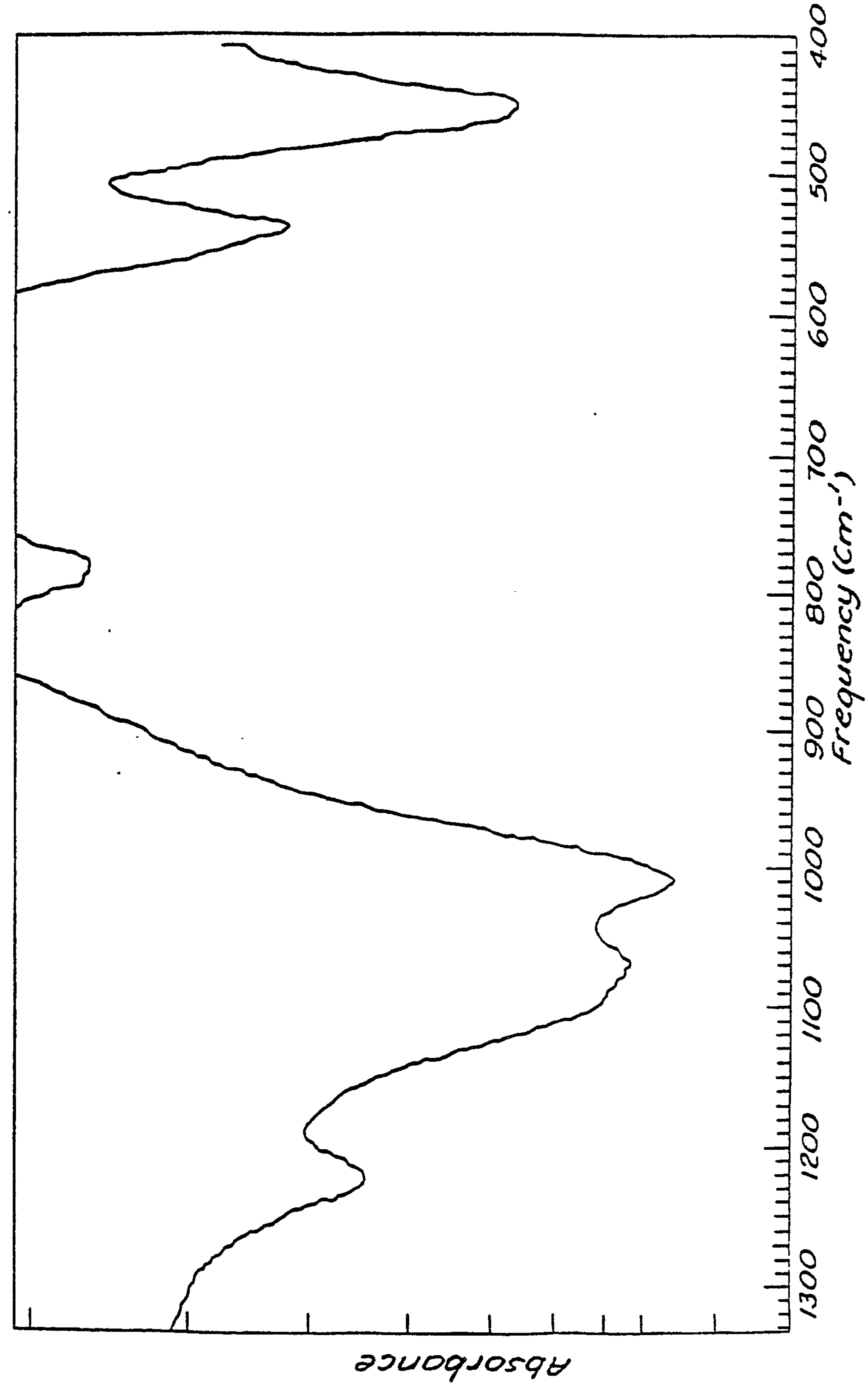
400
500
600
700
800
900
1000
1100
1200
1300
Frequency (cm$^{-1}$)
Absorbance

Fig. 1

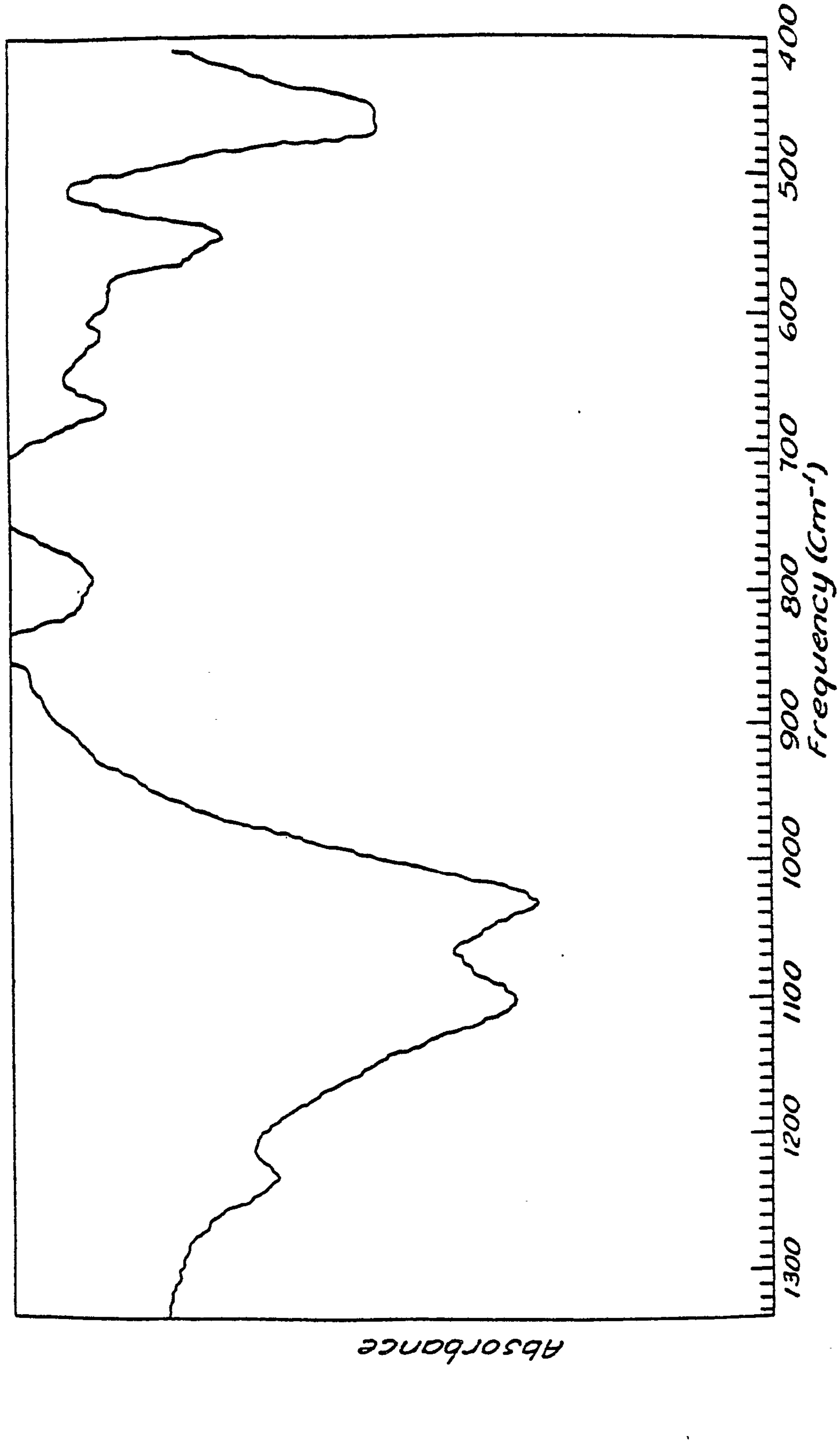
400
500
600
700
800
900
1000
1100
1200
1300
Frequency (Cm-1)
Absorbance

Fig. 2

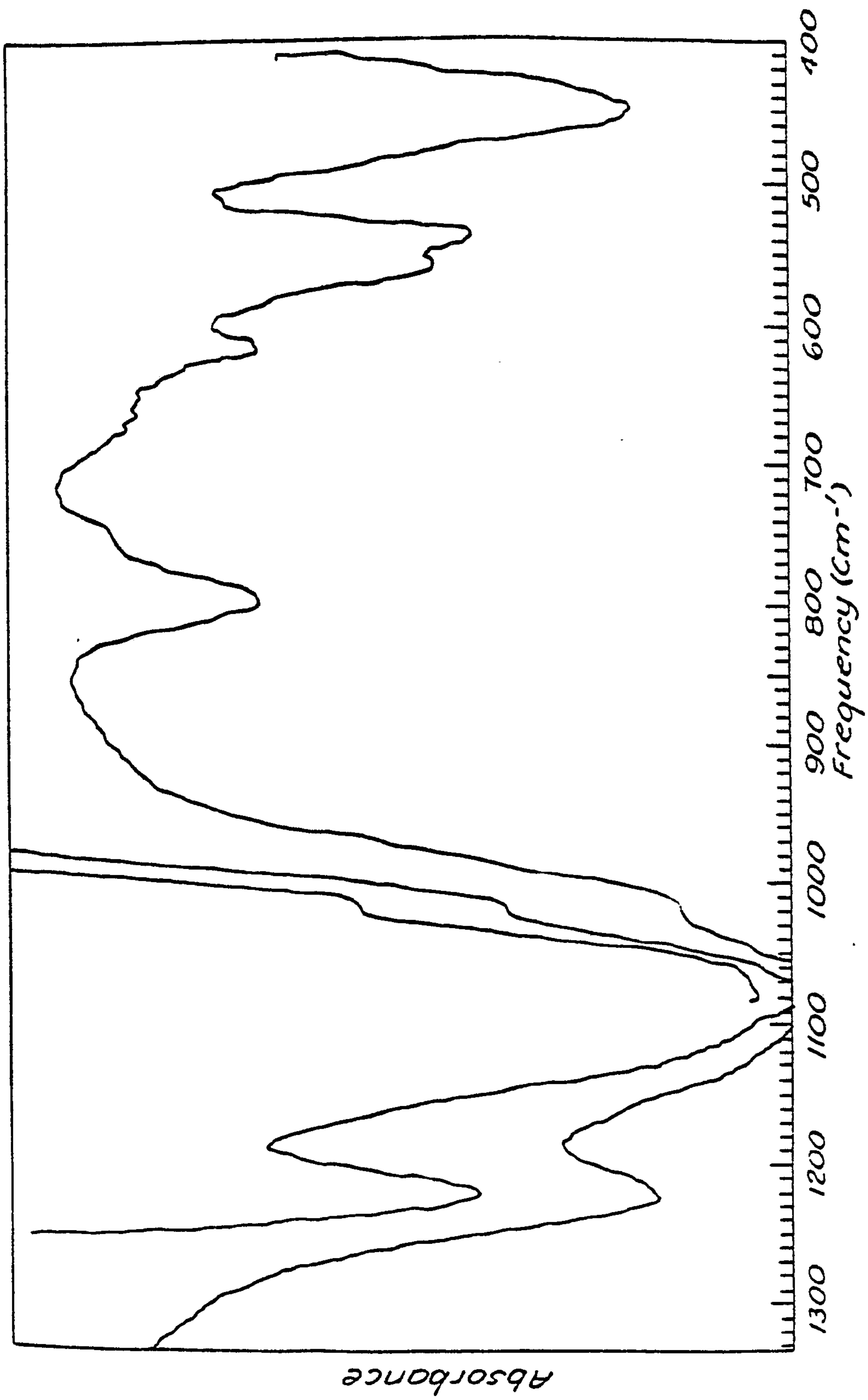
400
500
600
700
800
900
1000
1100
1200
1300
Frequency (cm-1)
Absorbance

Fig. 3